# EUROPEAN PATENT APPLICATION

(11) **EP 1 688 504 A1**
(43) Date of publication of application: **09.08.2006**
(21) Application number: 04799512.1
(22) Date of filing: 05.11.2004
(51) Int. Cl.: C12Q 1/02

(54) **SCREENING METHOD WITH THE USE OF TBK1 KNOCKOUT MOUSE**

(30) Priority: 10.11.2003 JP 2003380435
(71) Applicant: Japan Science and Technology Agency, Kawaguchi-shi, Saitama 332-0012 (JP)
(72) Inventor: AKIRA, Shizuo, Osaka 5690036 (JP)
(74) Representative: van Westenbrugge, Andries
(86) International application number: PCT/JP2004/016404
(87) International publication number: WO 2005/045064

(57) **Abstract**

The present invention is to provide a method for screening inductive promoting substances of anti-viral proteins such as IFN-β against LPS stimulation or viral infection by using TBK1 knockout mice, or the tissues or cells derived therefrom. The present invention also provides a method for screening substances promoting responses against LPS stimulation or viral infection comprising the steps of measuring/estimating the induction level of anti-viral proteins such as IFN-β against ligands recognized by TLR4 or substances containing thereof in mice wherein a part or a whole of TANK binding kinase-1 (TBK1) genes on its chromosome is deleted and is lacking the function to express TBK1 which is expressed in wild-type, or the tissues or cells derived therefrom; by using the mice, or the tissues or cells derived therefrom, a test substance, and the ligands recognized by TLR4 or substances containing thereof.

## Description

### Technical Field

The present invention relates to a method for screening inductive promoting substances of anti-viral proteins such as interferon β (IFN-β) for lipopolysaccharide (LPS) stimulation and/or viral infection with the use of TANK-binding kinase-1 (TBK1) knockout mice, or the tissues or cells derived therefrom.

### Background Art

It is known that Toll-like receptors (TLRs) are essential for recognizing invading pathogens and play an important role for a link between innate and adaptive immunity (e.g., see Annu. Rev. Immunol. 20:197-216, 2002; Annu. Rev. Immunol. 21:335-376, 2003). TLRs can recognize various bacterial components, such as triacylated lipopeptides recognized by TLR1/TLR2 heterodimer, diacylated lipopeptides recognized by TLR2/TLR6 heterodimer, lipopolysasccharide (LPS) recognized by TLR4, double-stranded RNA (dsRNA) recognized by TLR3, flagellin from bacterial flagella recognized by TLR5, and bacterial DNA containing the unmethylated CpG motif recognized by TLR9. At present, intracellular signaling pathways of TLRs are now being studied extensively (e.g., see Annu. Rev. Immunol. 20:197-216, 2002; Annu. Rev. Immunol. 21:335-376, 2003). The cytoplasmic region of each TLR contains a Toll/IL-1 receptor (TIR) domain that is common in both TLR and IL-1 receptor families. Signaling events of TLRs are initiated from the TIR domain by recruiting signaling molecules. MyD88 is a cytoplasmic molecule that contains both TIR and death domains, and functions as an adaptor protein for TLR and IL-1 receptor families. The TIR domain of MyD88 is required for forming a complex with TIR domain-containing receptor and an adaptor molecule. The death domain mediates interaction with other death domain-containing molecules such as IRAK1 and IRAK4. Although deficiency of MyD88 results in impaired production of proinflammatory cytokines in response to almost all TLR ligands, dsRNA (TLR3 ligand) and LPS (TLR4 ligand) induce interferon regulatory factor (IRF)-3 activation leading to IFN-β production in a MyD88-independent manner (e.g., seeJ.Immunol.167:5887-5894, 2001; Int. Immunol. 14:1225-1231, 2002; Nature Immunol. 3:392-8, 2002; J. Immunol. 169:6668-6672, 2002). Recent studies have revealed that the IRF-3 activation and IFN-β induction by dsRNA and LPS are mediated by another TIR-containing adaptor molecule, TRIF/TICAM-1 (e.g., see J.Immunol.169:6668-6672, 2002; Nature Immunol. 4:161-167, 2003; Science. 301:640-643, 2003; Nature. 10.1038/nature01889, 2003).

Molecular mechanisms of type I IFNs (IFN-α/β) induction in viral infection are studied in detail (e.g., see Annu. Rev. Microbiol. 55:255-81, 2001; Curr. Opin. Infect. Dis. 15:259-267, 2002; Curr. Opin. Immunol. 14:111-116, 2002). Induction of IFN-β is mainly regulated at transcriptional levels. Previous reports have shown that IFN-β expression is regulated by NF-κB, ATF-2/c-Jun, IRF-3 and IRF-7 (e.g., see Annu. Rev. Microbiol. 55:255-81, 2001; Curr. Opin. Infect. Dis. 15:259-267, 2002; Curr. Opin. Immunol. 14:111-116, 2002). Gene disruption studies have revealed that the important role of IRF-3 and IRF-7 in the transcription of type I IFN genes (e.g., see Immunity. 13:539-548, 2000; Biochem. Biophys. Res. Commun. 306:860-866, 2003) . During viral infection, IRF-3 is mainly responsible for initial activation of IFN-β gene (e.g., see EMBO J. 17:1087-1095, 1998), whereas IRF-7 expression depends on IFN-β. Thereby type I IFNs resulted in robust induction in an autocrine positive feedback manner (e.g., see Curr. Opin. Immunol. 14:111-116, 2002; Curr Opin Immunol. 15:52-58, 2003). IRF-3 is located in the cytoplasm of uninfected cells. After viral infection, IRF-3 is phosphorylated at multiple serine/thernine residues located in the C-terminal portion. Phosphorylated IRF-3 forms a homodimer that translocates into the nucleus and activates promoters containing the IRF-binding site, termed IRF-binding element/IFN-stimulated response element (IRF-E/ISRE).

Although the molecular mechanism to induce IRF-3 activation is poorly understood, recent reports suggested that IRF-3 is phosphorylated by inducible IKK (IKK-i) and TANK-binding kinase-1 (TBK1), two IκB kinase (IKK)-related kinases (e.g., see Science. 300:1148-1151, 2003; Nature Immunol. 4:491-496, 2003). IKK-i, also called IKK-ε, was originally identified as an LPS-inducible kinase (e.g., see Int. Immunol. 11:1357-1362, 1999; Mol. Cell. 5:513-522, 2000). TBK1 is also termed NF-κB activating kinase (NAK) or tumor necrosis factor (TNF) receptor-associated factor (TRAF)-2 associated kinase (T2K) (e.g., see EMBO J. 18:6694-6704, 1999; Nature. 404:778-782, 2000; EMBO J. 19:4976-4985, 2000). These two kinases share homology with IKK-α and IKK-β. Both kinases have been reported to interact with TRAF2 and the TRAF-binding protein, TANK/I-TRAF, and function upstream of IKK-α and IKK-β (e.g., see EMBO J. 18 : 6694-6704, 1999; Genes Cells. 5: 191-202, 2000) . Deficiency of TBK1 resulted in the embryonic lethality caused by massive liver degeneration, and TBK1-deficient (TBK1^{-/-}) embryonic fibroblasts (EFs) showed reduced expression of certain genes regulated by NF-κB (e.g., see EMBO J. 19:4976-4985, 2000). Thus, it is thought that these two IKK-related kinases have been implicated in NF-κB activation. Recently, two study groups have shown that IKK-i and TBK1 phosphorylate and activate IRF-3 and IRF-7, leading to transcription of IFN-β, RANTES (Regulated on activation, normal T cell expressed and secreted), and ISG54 in viral infection as well as in a TRIF-dependent signaling pathway (e.g., see Science. 300:1148-1151, 2003; Nature Immunol. 4:491-496, 2003) .

Further, it is also known that TBK1 (T2K) knockout mice die around embryonic day 14.5 (E14.5), and TBK1 (T2K) and TNF double-knockout mice grow in the same manner as wild-type mice do (see EMBO J. 19:4976-4985, 2000). Furthermore, lipopolysaccharide (LPS) existing mainly as outer membrane components of Gram-negative bacteria and also termed endotoxin, lipoteichoic acid (LTA) being cell wall components of Gram-positive bacteria, Mycobacterium tuberculosis lysate, Gram-positive bacteria cell wall fraction, and the like are known as ligands recognized by TLR4 (e.g., see Immunity. 11: 443-451, 1999; Int. Immunol. 12: 113-117, 2000).

### Disclosure of the Invention

The object of this invention is to provide a method for screening an inductive promoting substance of anti-viral proteins such as interferon β (IFN-β) for LPS stimulation and/or viral infection with the use of TBK1 knockout mice, or the tissues or cells derived therefrom.

The present inventors investigated the physiological roles of IKK-i and TBK1 by gene targeting. IKK-i^{-/-} cells showed normal gene induction in response to LPS. On the other hand, TBK1^{-/-} cells showed impaired IFN-β expression, while induction of proinflammatory cytokine mRNA was normal. NF-κB activation was induced in both mutant EFs in response to LPS, while ISRE-binding activity was not induced in TBK1^{-/-} cells. Moreover, expression of IFN-β and IFN-inducible genes was severely impaired in virus-infected TBK1^{-/-} cells. These results indicate that TBK1, but not IKK-i, is essential for IFN-β gene induction upon LPS stimulation and viral infection. The present invention has been thus completed based on these knowledge.

In other words, the present invention relates to (1) a method for screening a substance promoting a response to a ligand recognized by TLR4 or a substance containing thereof, wherein the response level to the ligand recognized by TLR4 or the substance containing thereof is measured/estimated in a mouse wherein a part or a whole of TANK binding kinase-1 (TBK1) gene is deleted, and a function of expressing TBK1 which is expressed in a wild-type is lacking, or in a tissue or cell derived therefrom, with the use of the mouse or the tissue or cell derived therefrom, a test substance, and the ligand recognized by TLR4 or the substance containing thereof.

Further, the present invention relates to (2) the method for screening a substance promoting a response to a ligand recognized by TLR4 or a substance containing thereof according to "1", wherein the measurement/estimation of the response level to the ligand recognized by TLR4 or the substance containing thereof is conducted by comparing/estimating with that of a littermate wild-type mouse as a control, (3) the method for screening a substance promoting a response to a ligand recognized by TLR4 or a substance containing thereof according to "1" or "2", wherein the cell derived from the mouse is an embryonic fibroblast derived from a TBK1 knockout mouse, (4) the method for screening a substance promoting a response to a ligand recognized by TLR4 or a substance containing thereof according to any one of "1"-"3", wherein the ligand recognized by TLR4 or the substance containing thereof is lipopolysaccharide (LPS), and (5) the method for screening a substance promoting a response to a ligand recognized by TLR4 or a substance containing thereof according to "4", wherein induction level of anti-viral protein against LPS stimulation is measured/estimated.

Furthermore, the present invention relates to (6) the method for screening a substance promoting a response to a ligand recognized by TLR4 or a substance containing thereof according to "5", wherein the anti-viral protein is interferon β (IFN-β), (7) the method for screening a substance promoting a response to a ligand recognized by TLR4 or a substance containing thereof according to "5", wherein the anti-viral protein is ISG54, IP-10 or IRG1, (8) the method for screening a substance promoting a response to a ligand recognized by TLR4 or a substance containing thereof according to "5", wherein the anti-viral protein is Mx2, IRF-7, interferon inducible GTPase, or ISG15, (9) the method for screening a substance promoting a response to a ligand recognized by TLR4 or a substance containing thereof according to "4", wherein induction level of ISRE-binding activity against LPS stimulation is measured/estimated, (10) the method for screening a substance promoting a response to a ligand recognized by TLR4 or a substance containing thereof according to any one of "1"-"3", wherein the ligand recognized by TLR4 or the substance containing thereof is a virus, (11) the method for screening a substance promoting a response to a ligand recognized by TLR4 or a substance containing thereof according to "10", wherein induction level of anti-viral protein against viral infection is measured/estimated, (12) the method for screening a substance promoting a response to a ligand recognized by TLR4 or a substance containing thereof according to "11", wherein the anti-viral protein is interferon β (IFN-β), and (13) the method for screening a substance promoting a response to a ligand recognized by TLR4 or a substance containing thereof according to "11", wherein the anti-viral protein against viral infection is ISG54, IP-10, IRG1 or RANTES.

### Brief Description of Drawings

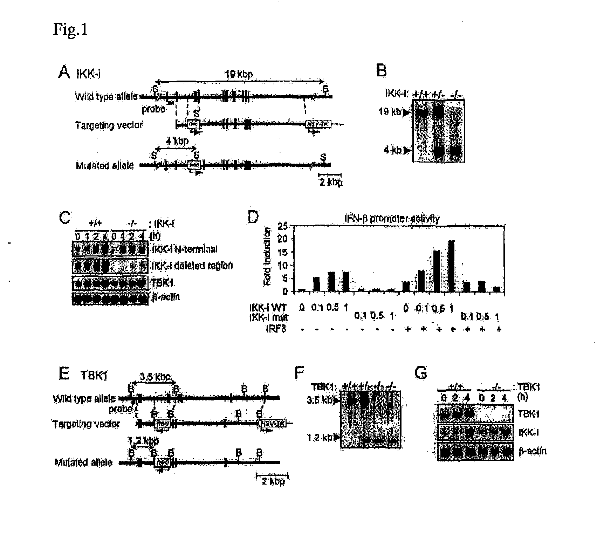

Figure 1 is a set of figures showing the generation of IKK-i^{-/-} and TBK1^{-/-} mice. (A) Structures of IKK-i gene, targeting vector, and predicted mutated allele are shown. Closed boxes (■) denote the coding exon. S is for restriction enzymes, SphI. (B) Results of Southern blot analysis of offsprings from the heterozygote intercrosses are shown. Genomic DNA was extracted from mouse tails, digested with SphI, eletrophoresed and hybridized with the radio-labeled probe indicated in (A). Results of Southern blotting showed a single 19 kbp band for wild-type (+/+), a single 4 kbp band for homozygotes mutants (-/-), and both bands for heterozygous mice (+/-). (C) Results of Northern blot analysis of thioglycollate-elicited peritoneal cells are shown. Thioglycollate-elicited peritoneal cells from wild-type and IKK-i^{-/-} mice were stimulated with 100 ng/ml of LPS for the indicated periods. Total RNA (10 µg) was electrophoresed, transferred onto a nylon membrane, and hybridized by using the IKK-i N-terminal fragment, the deleted region of IKK-i gene in targeting construct, or TBK1 cDNA fragment, as a probe. The same membrane was rehybridized with a β-actin probe. (D) Results of IFN-β promoter reporter assay is shown. HEK293 cells were transiently cotransfected with the indicated expression vectors and the IFN-β luciferase reporter vectors. 36 hr after transfection, luciferase activity in whole cell lysates was measured. (E) Structures of TBK1 gene, targeting vector, and predicted mutated allele are shown. Closed boxes (■) denote the coding exon. B is for restriction enzymes, BamHI. (F) Results of Southern blot analysis of EFs from the embryos of the heterozygote intercrosses are shown. Genomic DNA was extracted from EFs, digested with BamHI, electrophoresed, and hybridized with the radiolabeled probe indicated in (E). Results of Southern blotting showed a single 3.5 kbp band for wild-type (+/+), a single 1.2 kbp band for homozygotes mutants (-/-), and both bands for heterozygous mice (+/-). (G) Results of Northern blot analysis of EFs are shown. Total RNA was extracted from wild-type and TBK1^{-/-} EFs, stimulated with 1.0 µg/ml of LPS for the indicated periods, elctrophoresed, transferred onto a nylon membrane, and hybridized by using the IKK-i or TBK1 cDNA frangment as a probe. The same membrane was rehybridized with a β-actin probe.

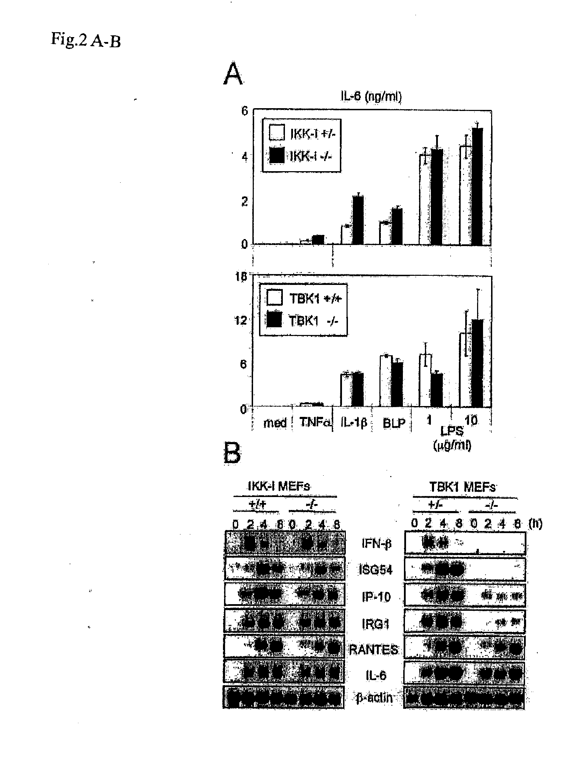

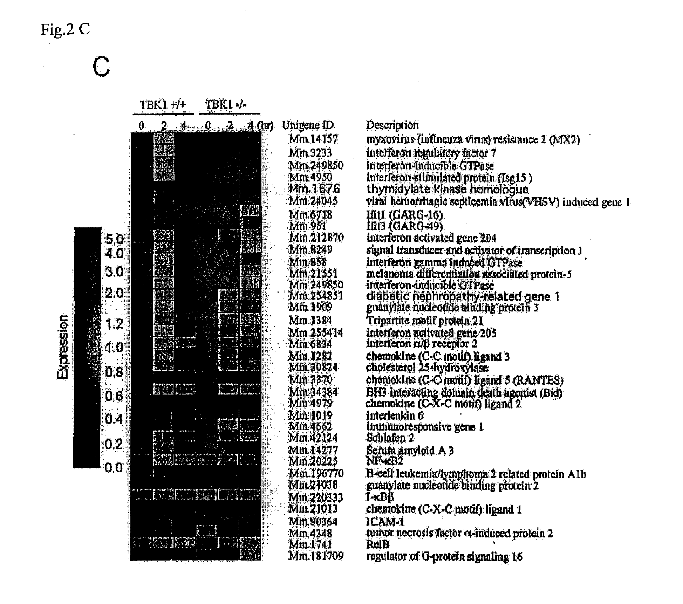

Figure 2 shows a set of figures showing the impaired induction of IFN-β and IFN-inducible genes in LPS-stimulated TBK1^{-/-}, but not IKK-i^{-/-} EFs . (A) IL-6 production by EFs is shown. Wild-type, IKK-i^{-/-} (top), or TBK1^{-/-} (bottom) EFs were stimulated with 10 ng/ml of TNF-α, 10 ng/ ml of IL-1β, 100 ng/ml of BLP, or the indicated concentrations of LPS for 24 hr. The concentration of IL-6 in the culture supernatants was measured by ELISA. Data are shown as means ± SD. (B) Gene induction in LPS-stimulated EFs is shown. Wild-type, IKK-i^{-/-} (left), or TBK1^{-/-} (right) EFs were stimulated with 1.0 µg/ml of LPS for the indicated periods. Total RNA was extracted and subjected to Northern blot analysis for the indicated genes. (c) Results of Microarray analysis of LPS-stimulated EFs are shown. Wild-type and TBK1^{-/-} EFs were stimulated with LPS, and RNA was collected at the indicated time points and used to conduct microarray analysis. Absolute expression was displayed by using Genespring software. The color code for signal strength is indicated on the left.

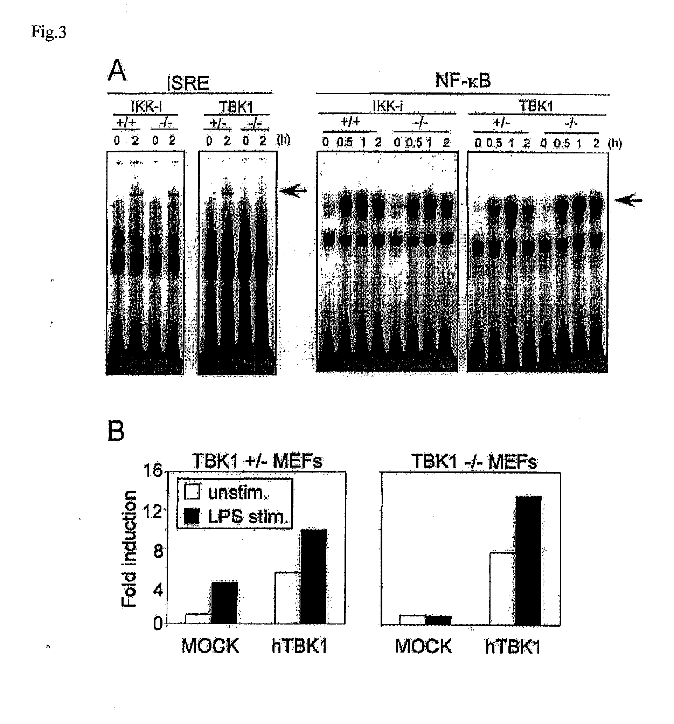

Figure 3 is a set of figures showing the necessity of TBK1 for LPS-induced ISRE-binding and IFN-β promoter activation. (A) Impaired ISRE-binding in TBK1^{-/-} EFs is shown. Wild-type, IKK-i^{-/-}, or TBK1^{-/-} EFs were stimulated with 0.1 µg/ml of LPS for the indicated periods, and NF-κB binding (right) and ISRE binding activities (left) were determined by EMSA. (B) It is shown that the expression of TBK1 restored the activation of IFN-β promoter in TBK1^{-/-} EFs. TBK1^{+/+} (WT) or TBK1^{-/-} EFs were cotransfected with human TBK1 (hTBK1) and the IFN-β promoter luciferase reporter. 24 hr after transfection, the cells were stimulated with LPS for additional 12 hr (LPS stim.) or unstimulated (unstim.) before measurement of luciferase activity.

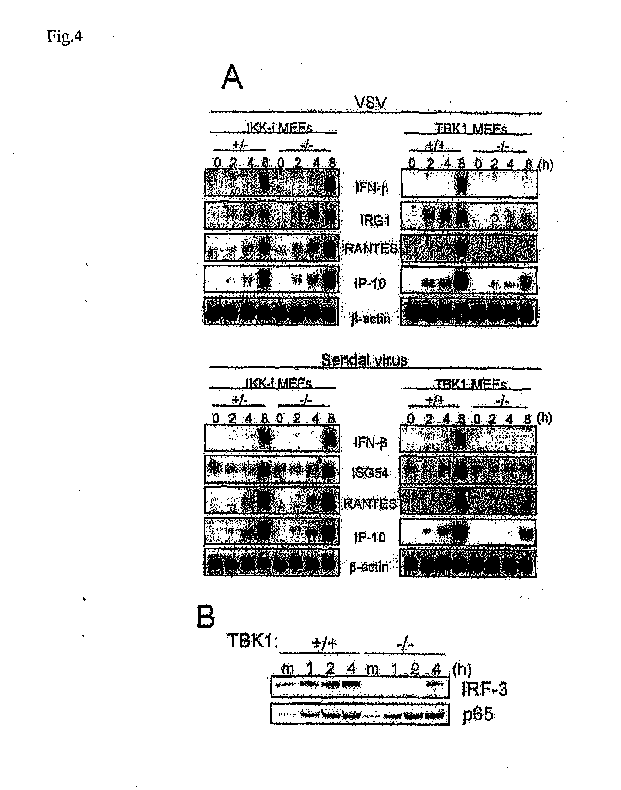

Figure 4 is a set of figures showing the necessity of TBK1 for IFN-inducible gene expression in virus-infected EFs. (A) Results of Northern blot analysis, which was conducted after infecting wild-type, IKK-i^{-/-} and TBK1^{-/-} EFs with recombinant VSV or SeV for the indicated periods to extract the total RNA, are shown. (B) Nuclear translocation of IRF-3 and NF-κB p65 in response to VSV infection is shown. EFs were infected with recombinant VSV for the indicated periods, and nuclear proteins were extracted, separated by SDS-PAGE, and blotted with anti-IRF-3 and NF-κB p65 antibodies.

### Best Mode of Carrying Out the Invention

In the present invention, as for a method for screening a inductive promoting substance of an anti-viral protein against LPS stimulation and/or viral infection, it is not particularly limited as long as it is a method for measuring/estimating the induction level of anti-viral protein to LPS stimulation and/or viral infection in a mouse wherein a part or a whole of TBK1 gene on its chromosome is deleted, and a function of expressing TBK1 expressed in a wild-type is lacking, or in a tissue or a cell derived from the mouse, with the use of the mouse or the tissue or cell derived from the mouse, a test substance and LPS and/or virus. As for the above mouse, TBK1 knockout mice or TBK1/TNF double knockout mice can be exemplified, however TBK1/TNF double knockout mice that grow in the same manner as wild-type mice do are preferable. Further, as for the tissues derived from the above mouse, spleens, lymph nodes, bone marrow, lungs and the like can be exemplified, and as for the cells derived from the above mouse, embryonic fibroblasts (EFs), macrophages, splenocytes, dendritic cells, lung fibroblasts and the like can be exemplified. As TBK1 knockout mice die around embryonic day 14.5 (E14.5), culturable immortalized cells such as embryonic fibroblasts and the like are preferable as cells derived from TBK1 knockout mice.

For example, the above TBK1 knockout mouse can be obtained in the following manners. TBK1 genes are obtained by amplifying mouse genomic library by PCR or the like, and the obtained gene fragments can be screened with a probe derived from mouse TBK1 gene. The screened TBK1 genes are subcloned with plasmid vectors and the like and can be identified by restriction enzyme mapping and DNA sequencing. Next, a whole or a part of the genes encoding TBK1 are replaced with pMC1 neo-gene cassette and the like, and targeting vectors are generated by introducing genes such as Diphtheria-toxin A fragment (DT-A) genes or herpes simplex virus thymidine kinase (HSV-tk) genes to the 3'end.

The generated targeting vectors are linearized and introduced into ES cells by electroporation and the like for homologous recombination, and the ES cells having performed homologous recombination by antibiotics such as G418 or Gancyclovir (GANC) are selected from the homologous recombinants. Moreover, it is preferable to confirm whether the selected ES cells were the desired recombinants by Southern blot method or the like. Clones of the confirmed ES cells are microinj ected into mouse blastocysts, and the mouse blastocysts are returned to recepient mice to generate chimeric mice. The chimeric mice are intercrossed with wild-type mice to obtain heterozygous mice (F1 mice: +/-), and by breeding the heterozygous mice, TBK1 knockout (TBK1^{-/-}) mice of the present invention can be generated. Moreover, as for a method for confirming whether TRAM has been generated in TBK1 knockout mice, for example, there is a method for examining RNA isolated from the mice obtained by the above-mentioned method, by Northern blot method or the like, or for examining TBK1 expression in these mice by Western blot method or the like.

Furthermore, it can be confirmed whether the generated TBK1 knockout mice are non-responsive to ligands recognized by TLR4 or substances containing thereof, for example as follows; by administering LPS being a component of Gram-negative bacteria cell wall, virus and the like to TBK1 knockout mice by intravenous injection or the like, and then by measuring the biological activity of endotoxin such as pyrexia, shock, reduction of leukocytes or platelets, hemorrhagic necrosis in bone marrow, hypoglycemia, IFN induction, activation of B-lymphocytes (bone marrow-derived immune response cells);or by measuring the expression of anti-virus proteins such as IFN-β, in TBK1 knockout mouse embryonic fibroblasts, in the presence of bacterial LPS, or lipoteichoic acid being component of Gram-positive bacteria, Mycobacterium tuberculosis lysate, virus, and the like. As for the above virus, as long as virus components include ligands recognized by TLR4, it is not particularly limited, and for example, vesicular stomatitis virus (VSV), Sendai virus (SeV), mouse mammary tumor virus (MMTV), RS virus (RSV) and the like can be exemplified.

In the present invention, as for the method for screening substances promoting responses to ligands recognized by TLR4 or substances containing thereof, specifically, the following can be exemplified; a method for measuring/estimating the response level for LPS stimulation or viral infection in cells derived from these knockout mice comprising the steps of administering LPS, infecting with viruses, and administering peptides, nucleic acid, low molecular compounds, test substances such as naturally-derived extracts before or after or at the same time as LPS stimulation or viral infection, to the cells derived from the above mentioned TBK1 knockout or TBK1/TNF double-knockout mice; and a method for measuring/estimating the response level for LPS stimulation or viral infection in cells derived from these knockout mice comprising the steps of stimulating with LPS, infecting with viruses, and co-existing peptides, nucleic acid, low molecular compounds, test substances such as naturally-derived extracts before or after or at the same time as LPS administration (stimulation) or viral infection, to the above mentioned TBK1 knockout or TBK1/TNF double-knockout mice. Furthermore, when measuring/estimating the response levels to ligands recognized by TLR4 or substances containing thereof, it is preferable to be able to compare/estimate with the measurements of the littermates of wild-type mice as control, so that individual variety can be eliminated.

When the ligands recognized by TLR4 are LPS, it is preferable to measure/estimate the induction level of anti-virus proteins against LPS stimulation and ISRE binding activity, and as for the above anti-virus proteins, IFN-β; ISG54, IP-10 (IFN-γ-Inducible Protein10) and IRG1 induced by IFN-β; Mx2, IRF-7, interferon inducible GTPase, ISG15 and the like can be specifically exemplified. When the substances containing ligands recognized by TLR4 are viruses, it is preferable to measure/estimate induction level of anti-virus proteins against viral infection, and as for the above anti-virus proteins, IFN-β; ISG54, IP-10, IRG1 and RANTES induced by IFN-β; and the like can be specifically exemplified.

Promoting substances obtained by the method for screening substances promoting the responses to ligands recognized by TLR4 of the present invention or substances containing thereof are useful to elucidate the mechanism in signaling by LPS stimulation or viral infection *in vivo,* and promoting substances can possibly be used as preventive/treating agents for bacterial and viral infections. When the above-mentioned promoting substances wherein preventive/treating effect for bacterial and viral infections can be expected as pharmaceuticals are used, various prescribed compounds such as pharmaceutically acceptable normal carriers, bonding agents, stabilizing agents, excipients, diluents, pH buffer agents, disintegrators, solubilizers, dissolving adjuvants, isotonic agents can be added. Further, in a preventing or treating method by using these pharmaceuticals, appropriate dosage suited to sex, body weight and symptom of patients can be administered orally or parenterally. In other words, it can be administered orally in normal dosage forms, for example in forms of powder, granule, capsules, syrup, suspension and the like, or parenterally for example in forms of solution, emulsion, suspension and the like by injection, and moreover, it can be administered through nostril in form of spray.

The present invention will be explained specifically in the following by referring to the examples, but the technical scope of the present invention will not be limited to these.

### Example 1

### [Materials and Methods]

### (Cells, virus, and Reagents)

Thioglycollate-elicited peritoneal cells were collected 3 days after intraperitoneal injection of 2 ml of 4% thioglycollate. Embryonic fibroblasts (EFs) were prepared from embryonic day 12.5 embryos as described previously (J. Immunol. 167:5887-5894, 2001). Recombinant vesicular stomatitis virus (VSV) was provided by Dr. Yoshiharu Matsuura and Dr. Takayuki Abe (Osaka University). Sendai virus (SeV) was provided by Dr. Tatsuo Shiota (Osaka University) . EFs were infected with 1×10⁹ RNA copies/ml of VSV or multiplicity of infection (MOI) 10 of SeV. LPS from *Salmonella Minnesota* (S. minnesota) Re-595 was purchased from Sigma-Aldrich. Synthetic Pam₃CSK₄ (bacterial lipopeptide, BLP) was obtained from Boehringer Mannheim. TNF-α and IL-1β were purchased from Genzyme.

### (Plasmids)

To construct the expression vectors for wild-type IKK-i and mutant IKK-i derived from the IKK-i mutated allele, RT-PCR products of IKK-i cDNA from wild-type and IKK-i^{-/-} EFs were sequenced and cloned into pFLAG-CMV2 vector (Sigma-Aldrich). Human IRF-3 expression vector was constructed by ligation of human IRF-3 cDNA into pFLAG-CMV2 vector. RT-PCR products of TBK1 cDNA from human placenta cDNA library were sequenced, and the expression vector for human TBK1 (hTBK1) was constructed by ligation of human TBK1 cDNA into the pEF-BOS vector.

### (Generation of IKK-i^{-/-} and TBK1^{-/-} mice)

Genomic DNA fragments encoding IKK-i and TBK1 were screened from the 129/Sv mice genomic library and characterized by restriction enzyme mapping and sequencing analysis. The targeting vectors were designed by replacing both 1.0 kbp fragment of IKK-i gene and 1.5 kbp fragment of TBK1 gene with a neomycin resistant gene cassette. A herpes simplex virus thymidine kinase gene driven by MC1 promoter was used for negative selection. These targeting vectors were transfected into embryonic day 14.1 ES cells. Targeted ES cells were identified among G418 and ganciclovir doubly resistant clones by PCR and Southern blotting and subsequently injected into C57BL/6 blastocysts. The obtained male chimeric mice were bred with C57BL/6 female mice. The F1 offsprings were then intercrossed with each other to generate homozygotes for each mutated allele.

### (Northern blot analysis)

Total RNA was isolated by using Sepazol-RNA I (Nacalai Tesque), electrophoresed, and transferred onto nylon membranes. Hybridization was performed with the indicated cDNA probes as described previously (J. Immunol. 167:5887-5894, 2001). All cDNA probes were obtained from subtractive screening as described previously (J. Immunol. 167:5887-5894, 2001).

### (Electrophoretic Mobility Shift Assay (EMSA))

EFs (1×10⁶ cells) from wild-type, IKK-i^{-/-} and TBK1^{-/-} embryos were stimulated with 1.0 µg/ml of LPS for the indicated periods. Nuclear extracts were prepared and incubated with a radio-labeled oligonucleotide probe containing NF-κB binding sites or IFN-stimulated regulatory element (ISRE), and visualized by autoradiography as described previously (J. Immunol. 167:5887-5894, 2001).

### (Western blot analysis)

Nuclear extracts were separated by SDS-PAGE and transferred onto PVDF membranes. The membrane was blotted with anti-IRF-3 antibody (Science. 301:640-643, 2003) and anti-NF-κB p65 antibody (Santa Cruz Biotechnology). The membrane-bound antibodies were visualized with horseradish peroxidase-conjugated antibody to rabbit IgG (Amersham Bioscience) by using the ECL system (PerkinElmer Life Sciences).

### (Reporter assay)

Human embryonic kidney 293 cells were seeded onto 24-well plate and transiently transfected by the indicated expression vector together with a reporter plasmid by using Lipofectoamin 2000 reagent (Invitrogen) . EFs seeded onto a 6-well plate were transiently transfected with a empty pEF-BOS vector (MOCK) or pEF-BOS-human TBK1 together with the IFN-β promoter reporter plasmid by using FuGENE 6 transfection reagent (Roche Diagnostics). 24 hr after the transfection, the cells were stimulated with 10 µg/ml of LPS for an additional 12 hr. Luciferase activity of whole cell lysates was measured by using a *Dual*-luciferase reporter assay system (Promega). A *Renilla*-luciferase reporter gene (Promega) was used as an internal standard.

### (Microarray analysis)

Total RNA was extracted from EFs stimulated with LPS for 2 or 4 hr or unstimulated, and subjected to synthesis of cRNA probe. Preparation of cRNA, hybridization, and scanning of the microarray were performed according to the manufacture's instruction. Affymetrix microarray MG U74A version 2 (Affymetrix) was used for the analysis. Data analysis was performed with Microarray Suite version 5.0 software (Affymetrix) and GeneSpring software (Silicon Genetics).

### Example 2

### [Results]

### (Generation of IKK-i^{-/-} and TBK1^{-/-} mice)

To investigate the physiological role of IKK-i and TBK1, IKK-i^{-/-} and TBK1^{-/-} mice were generated by gene targeting. Targeting vectors used to generate IKK-i^{-/-} mice were constructed to replace exons 7 and 8 of the IKK-i gene encoding a part of the second kinase domain with a neomycin resistant gene cassette (Fig. 1A). IKK-i^{-/-} mice were born at the expected Mendelian ratio, were fertile, and appeared to be healthy (Fig. 1B). By using an N-terminal fragment of the IKK-i gene as a probe, IKK-i transcripts in thioglycollate-elicited peritoneal cells from homozygous mutant mice were detected (Fig. 1C). However, sequencing analysis showed that the IKK-i mRNA from the mutant allele lacked the targeted exons, contained a premature stop codon, and generated a truncated protein containing only one of the two kinase domains (data not shown) . Transient transfection assay was used to estimate biological activity of wild-type and mutant IKK-i proteins (Fig. 1D). The wild-type IKK-i protein, but not the mutant, induced activation of IFN-β promoter in a dose-dependent manner. Therefore, it was concluded that the mutant IKK-i protein does not have biological activity, at least for IFN-β induction.

The targeting vector used to generate TBK1^{-/-} mice was constructed to replace exon 9 of the TBK1 gene with a neomycin-resistant gene cassette (Fig. 1E). Mice heterozygous for TBK1 were born and appeared to be healthy. However, TBK1^{-/-} mice died around embryonic day 14.5 (E14.5), as reported previously (EMBO J. 19:4976-4985, 2000). Embryonic fibroblasts (EFs) were obtained from embryos at E12.5 (Fig. 1F) and TBK1 mRNA expression was examined. In wild-type EFs, TBK1 mRNA was detected, which increased after LPS stimulation. However, in TBK1^{-/-} EFs, TBK1 mRNA was not detected at all, whereas IKK-i gene was normally induced (Fig. 1G).

### (Induction of IL-6 production in IKK-i^{-/-} and TBK1^{-/-} cells)

IKK-i and TBK1 are implicated in NF-κB activation. As IL-6 production is dependent on NF-κB, the present inventors measured the IL-6 production in EFs in response to various stimulations known to activate NF-κB. EFs were stimulated with TNF-α, IL-1β, the TLR2 ligand Pam3CSK4 (bacterial lipopeptides, BLP), and the TLR4 ligand LPS. As it is shown in Fig. 2A, IKK-i^{-/-} and TBK1^{-/-} EFs produced similar levels of IL-6, compared with wild-type cells in response to all stimulants tested.

### (Induction of a set of IFN-inducible genes in response to LPS in TBK1^{-/-} EFs)

LPS stimulation induces IRF-3 activation, which leads to the induction of IFN-β and a set of IFN-inducible genes (J. Immunol. 167:5887-5894, 2001; Nakaya, T, M. Sato, N. Hata, M. Asagiri, H. Suemori, S. Noguchi, N. Tanaka, and T. Taniguchi. Gene induction pathways mediated by distinct IRFs during viral infection. 2001. Biochem. Biophys. Res. Commun. 283:1150-1156). Therefore, the expression of these IRF-3 regulated genes was examined by Northern blot analysis (Fig. 2B). In IKK-i^{-/-} EFs, enhanced expression of IFN-β and IL-6 genes was observed in response to LPS. Furthermore, expression of IFN-inducible genes such as ISG54, IP-10, IRG1 and RANTES were also upregulated in LPS-stimulated IKK-i^{-/-} EFs. These responses were also intact in bone marrow-derived dendritic cells, and thioglycollate-elicited peritoneal cells from IKK-i^{-/-} mice (data not shown). In contrast, mRNA induction of IFN-β, ISG54, IP-10 and IRG1 was severely impaired in TBK1^{-/-} EFs, whereas mRNA induction of RANTES and IL-6 was similar to that of wild-type cells (Fig. 2B).

Next, DNA microarray analysis of LPS-stimulated EFs was conducted. Wild-type and TBK1^{-/-} EFs were stimulated with LPS for 2 or 4hr. Genes induced by wild-type EFs are showed in Fig. 2C. IFN-inducible genes such as Mx2, IRF-7, interferon inducible GTPase, and ISG15 were not induced in TBK1^{-/-} EFs. In contrast, TBK1^{-/-} cells showed normal induction of MyD88-dependent genes such as IL-6, ICAM-1 and IκB-β (Fig. 2C) . EMSA analysis revealed that normal NF-κB activation was observed in both IKK-i^{-/-} and TBK1^{-/-} cells similarly to that of wild-type cells (Fig. 3A). On the other hand, ISRE binding activity was not induced in TBK1^{-/-} cells, but observed in both wild-type and IKK-i^{-/-} cells (Fig. 3A).

To confirm that TBK1 is required for the IFN-β induction, it was examined whether re-constitution of TBK1 in TBK1^{-/-} cells could restore IFN-β promoter activation upon LPS stimulation (Fig. 3B). LPS activated the IFN-β promoter in mock-transfected wild-type EFs, but not in TBK1^{-/-} EFs. However, transient expression of human TBK1 conferred the ability to activate the IFN-β promoter in LPS stimulated TBK1^{-/-} EFs. Thus, these results suggest that TBK1 is essential for the TLK4-mediated IFN-β expression.

### (TBK1 is an essential molecule for IFN-β gene induction in viral infection)

Viral infection induces phosphorylation and dimerization of IRF-3 and leads to the induction of IFN-β and other anti-viral molecules (Lin, R, C. Heylbroeck, P. Genin, P. M. Pitha, J. Hiscott. 1999. Essential role of interferon regulatory factor 3 in direct activation of RANTES chemokine transcription. Mol. Cell Biol. 19:959-966; Genin, P., M. Algarte, P. Roof, R. Lin, and J. Hiscott. 2000. Regulation of RANTES chemokine gene expression requires cooperativity between NF-κB and IFN-regulatory factor transcription factors. J. Immunol. 164:5352-5361). Therefore, it was examined which of the IKK-i and TBK1 mediates virus-induced gene expression (Fig. 4A). EFs were infected with vesicular stomatitis virus (VSV) or Sendai virus (SeV), and mRNA expression was examined by Northern blot analysis. In IKK-i^{-/-} EFs, induction of mRNA for IFN-β, RANTES and IP-10 was similar to that of wild-type cells in response to VSV or SeV viral infection. On the other hand, in TBK1^{-/-} cells, mRNA expression of IFN-β was not observed, and mRNA expression of RANTES and IP-10 was significantly diminished compared with that in wild-type cells. To investigate IRF-3 activation, the nuclear proteins prepared from VSV-infected wild-type and TBK1^{-/-} EFs were immunoblotted to detect IRF-3. As it is shown in Fig. 4B, accumulation of IRF-3 and NF-κB p65 in the nucleus was observed in wild-type cells. The accumulation of IRF-3 was impaired in TBK1^{-/-} cells, whereas NF-κB p65 translocation was not affected. These results suggest that TBK1 is an essential molecule for IRF-3 activation, which leads to mRNA induction of IFN-β and other anti-viral molecules in viral infection. Notably, induction of RANTES transcription was impaired in virus infected TBK1^{-/-} EFs, on the other hand, when stimulated with LPS, mRNA of RANTES was clearly induced.

### [Discussion]

In the above Example 2, it has been demonstrated that TBK1 is essential for the induction of a set of IFN-induced genes including IFN-β. These genes are regulated in a MyD88-independent but TRIF-dependent manner and are activated by transcription factor IRF-3 (see J. Immunol. 167:5887-5894, 2001; Science. 301:640-643, 2003; Nature. 10.1038/nature01889, 2003). Furthermore, it has been reported that TBK1 interacts with TRIF and phosphorylatesIRF-3 (see Science. 300:1148-1151, 2003; Nature Immunol. 4:491-496, 2003), and suggested that TBK1 is an essential molecule for the TLK4-TRIF-IRF3 signaling pathway. Consistent with these data, induction of ISRE binding activity was abolished in TBK1^{-/-} cells. In addition, mRNA induction of IFN-β, RANTES and IP-10 in viral infection was abolished in TBK1^{-/-} cells. IKK-i^{-/-} cells, on the other hand, showed normal levels of gene induction in response to both LPS and viral infections. Thus, although IKK-i and TBK1 have shown similar functions *in vitro* studies (see Science. 300:1148-1151, 2003; Nature Immunol. 4:491-496, 2003; Int. Immunol. 11:1357-1362, 1999; Mol. Cell. 5:513-522, 2000; Nature. 404:778-782, 2000), data in the above Example 2 have indicated that these two kinases have different functions in LPS stimulation and viral infection.

IKK-i and TBK1 were primarily identified as IKK-α and IKK-β related molecules. Overexpression of IKK-α, IKK-β IKK-i, or TBK1 leads to NF-κB activation. However, IKK-i and TBK1 are different from IKK-α and IFN-β in how they activate NF-κB. IKK-α and IKK-β phosphorylate both serines 32 and 36, whereas IKK-i and TBK1 phosphorylate only serine 36 (see Int. Immunol. 11:1357-1362, 1999; Mol. Cell. 5:513-522, 2000; EMBO J. 18:6694-6704, 1999; Nature. 404:778-782, 2000). Furthermore, recent reports have shown that IKK-i and TBK1 interact with and phosphorylate TANK/I-TRAF (see EMBO J. 18:6694-6704, 1999; Genes Cells. 5:191-202, 2000), and the association of TANK with NEMO/IKK-γ and IKK-α/β complex is significantly increased in the presence of IKK-i or TBK1, suggesting that IKK-i and TBK1 are involved in NF-κB activation upstream of IKK-α/β (see Chariot A, Leonardi A, Muller J, Bonif M, Brown K, Siebenlist U. 2002. Association of the adaptor TANK with the IkB kinase (IKK) regulator NEMO connects IKK complexes with IKKe and TBK1 kinases. J. Biol. Chem. 277:37029-37036. First published on July 19, 2002; 10.1074/jbc.M205069200). However genetic knockout study revealed that NF-κB DNA-binding activity is upregulated upon either TNF-α or IL-1 stimulation in TBK1^{-/-}cells (see Nature. 404:778-782, 2000). It was also identified that upregulation of NF-κB DNA-binding activity in TBK1^{-/-} EFs was comparable to that observed in wild-type cells in response to LPS as well as TNF-α and IL-1β (Fig. 3A). In addition, IL-6 production, which depends on NF-κB activation, was normal in TBK1^{-/-} cells. Although it has been previously reported that IKK-i^{-/-} cells show reduction of IL-6 production in response to LPS (see Kravchenko, V. V., J. C. Mathison, K. Schwamborn, F. Mercurio, and R. J. Ulevitch. 2003. IKKi/IKKe plays a key role in integrating signals induced by pro-inflammatory stimuli. J. Biol. Chem. 278:26612-26619. First published on May 6, 2003; 10.1074/jbc. M303001200), similar NF-κB activation and IL-6 production were observed in LPS-stimulated IKK-i^{-/-} EFs in the experiments conducted by the present inventors. Therefore, it was suggested that IKK-i and TBK1 are not necessary for NF-κB activation leading to proinflammatory cytokine production. Nevertheless, the possibility that IKK-i and TBK1 compensate for NF-κB activation with each other cannot be ruled out.

It has known that TLR3 or TLR4 stimulation to macrophages leads to the immediate upregulation of IFN-β, IP-10, and RANTES genes in an IRF-3-dependent manner (see Doyle, S., S. Vaidya, R. O'Connell, H. Dadgostar, P. Dempsey, T. Wu, G. Rao, R. Sun, M. Haberland, R. Modlin, and G. Cheng. 2002. IRF3 mediates a TLR3/TLR4-specific antiviral gene program. Immunity. 17:251-263). As it is shown in the above Example 2, it has been revealed that induction of IFN-β, IP-10, and RANTES transcription was abolished in TBK1^{-/-} EFs infected with VSV or SeV. However, RANTES mRNA was induced in TBK1^{-/-} EFs in response to LPS. Although RANTES mRNA induction appeared to be slightly reduced in TBK1^{-/-} EFs compared with that in wild-type cells, this reduction may be due to the lack of secondary upregulation by autocrine production of IFN-β. At present, the molecular mechanism of this discrepancy is not understood, but these results suggest that RANTES mRNA expression is regulated differently from the IFN-β and IP-10 expression, depending on the cell type or the stimulus applied.

### Industrial Applicability

In conclusion, as the present invention revealed that TBK1, but not IKK-i, is an essential component for the IFN-β gene induction in response to LPS and viral infection, IFN-β expression can be changed without affecting the induction of proinflammatory cytokines by controlling TBK1 activity. With the present invention, new therapeutic treatments for septic shock and viral infection can be obtained.

## Claims

1. A method for screening a substance promoting a response to a ligand recognized by TLR4 or a substance containing thereof, wherein the response level to the ligand recognized by TLR4 or the substance containing thereof is measured/estimated in a mouse wherein a part or a whole of TANK binding kinase-1 (TBK1) gene is deleted, and a function of expressing TBK1 which is expressed in a wild-type is lacking, or in a tissue or cell derived therefrom, with the use of the mouse or the tissue or cell derived therefrom, a test substance, and the ligand recognized by TLR4 or the substance containing thereof.

2. The method for screening a substance promoting a response to a ligand recognized by TLR4 or a substance containing thereof according to claim 1, wherein the measurement/estimation of the response level to the ligand recognized by TLR4 or the substance containing thereof is conducted by comparing/estimating with that of a littermate wild-type mouse as a control.

3. The method for screening a substance promoting a response to a ligand recognized by TLR4 or a substance containing thereof according to claim 1 or 2, wherein the cell derived from the mouse is an embryonic fibroblast derived from a TBK1 knockout mouse.

4. The method for screening a substance promoting a response to a ligand recognized by TLR4 or a substance containing thereof according to any one of claims 1-3, wherein the ligand recognized by TLR4 or the substance containing thereof is lipopolysaccharide (LPS).

5. The method for screening a substance promoting a response to a ligand recognized by TLR4 or a substance containing thereof according to claim 4, wherein induction level of anti-viral protein against LPS stimulation is measured/estimated.

6. The method for screening a substance promoting a response to a ligand recognized by TLR4 or a substance containing thereof according to claim 5, wherein the anti-viral protein is interferon β (IFN-β).

7. The method for screening a substance promoting a response to a ligand recognized by TLR4 or a substance containing thereof according to claim 5, wherein the anti-viral protein is ISG54, IP-10 or IRG1.

8. The method for screening a substance promoting a response to a ligand recognized by TLR4 or a substance containing thereof according to claim 5, wherein the anti-viral protein is Mx2, IRF-7, interferon inducible GTPase, or ISG15.

9. The method for screening a substance promoting a response to a ligand recognized by TLR4 or a substance containing thereof according to claim 4, wherein induction level of ISRE-binding activity against LPS stimulation is measured/estimated.

10. The method for screening a substance promoting a response to a ligand recognized by TLR4 or a substance containing thereof according to any one of claims 1-3, wherein the ligand recognized by TLR4 or the substance containing thereof is a virus.

11. The method for screening a substance promoting a response to a ligand recognized by TLR4 or a substance containing thereof according to claim 10, wherein induction level of anti-viral protein against viral infection is measured/estimated.

12. The method for screening a substance promoting a response to a ligand recognized by TLR4 or a substance containing thereof according to claim 11, wherein the anti-viral protein is interferon β (IFN-β).

13. The method for screening a substance promoting a response to a ligand recognized by TLR4 or a substance containing thereof according to claim 11, wherein the anti-viral protein against viral infection is ISG54, IP-10, IRG1 or RANTES.
